# EUROPEAN PATENT SPECIFICATION

(11) **EP 4 135 648 B1**
(45) Date of publication and mention of the grant of the patent: **04.06.2025**
(21) Application number: 21715658.7
(22) Date of filing: 01.04.2021
(51) Int. Cl.: A61K 8/02, A61K 8/36, A61K 8/86, A61K 8/60, A61K 8/49, A61K 8/39, A61K 8/92, A61Q 19/10, A61Q 5/02

(54) **HYDRATABLE COSMETIC COMPOSITION**
HYDRATISIERBARE KOSMETISCHE ZUSAMMENSETZUNG
COMPOSITION COSMÉTIQUE HYDRATABLE

(30) Priority: 14.04.2020 EP 20169457
(43) Date of publication of application: 22.02.2023
(73) Proprietor: Unilever IP Holdings B.V., 3013 AL Rotterdam (NL); Unilever Global IP Limited, Wirral, Merseyside CH62 4ZD (GB)
(72) Inventor: BEKTO, Hasiba, Trumbull, CT Connecticut 06611 (US); CROTTY, Brian, Andrew, Trumbull, CT Connecticut 06611 (US); MOADDEL, Teanoosh, Trumbull, CT Connecticut 06611 (US); WEST, Alan, Michael, Trumbull, CT Connecticut 06611 (US)
(74) Representative: James, Helen Sarah
(86) International application number: PCT/EP2021/058783
(87) International publication number: WO 2021/209274

(56) References cited:
- WO-A1-03/045347
- WO-A1-2004/089330
- WO-A1-99/49878
- US-A1- 2016 263 014

## Description

### Technical Field

The present invention is in the field of personal care compositions. More particularly, the present invention relates to a rapidly hydratable cosmetic composition precursor, as defined in claim 1, for customization comprising of partially neutralized fatty acid and high HLB surfactant. The invention also relates to end-use compositions made from the hydratable composition of the invention.

### Background

Cosmetic compositions have traditionally been categorized as targeting defined benefit platforms, including dry skin, reactive skin, acne and anti-aging. However, in recent years, as consumers become increasingly cognizant that an individual's skin is unique from that of another, there is a growing demand and need for bespoke personal care products. Many of these bespoke cosmetic products are either formulated specifically for a consumer's disclosed needs or capable of being further customized by the consumers themselves after the base product has been formulated.

Customizing cosmetic compositions for individual consumers is faced with various challenges. Particularly noteworthy obstacles include the additional amount of time needed to create a personalized product for an individual from start to finish and the potential waste resulting from the creation of a bespoke product, since, in theory, that specific formula was catered to a single consumer's unique skin needs.

Therefore, the present inventors have recognized a need to develop a cosmetic composition precursor that is rapidly hydratable and conducive to creating a personalized end-product. This enables the consumer to create the exact amount of product that they expect they could use in a certain period and to customize the base exactly to their liking at any location of preference. Furthermore, the cosmetic composition precursor is sustainable and environmentally friendly, as it has the potential to be used as a refill, shipped in the absence of added water, and packaged with reduced plastic use. This invention is directed to a cosmetic composition precursor having partially neutralized fatty acid and high HLB surfactant as well as end use compositions made from the same.

### Additional Information

Efforts have been disclosed for creating cosmetic compositions.

U.S. Patent No. 4992477 relates to a skin moisturizing composition and method of preparing the same comprising an emulsion formed by combining an oil phase comprising oil and a non-ionic, high HLB surfactant and an aqueous phase with thickening agent and water.

U.S. Patent No. 5658575 discloses a cosmetic or dermatological composition comprising an oil-in-water emulsion containing oily globules coated with a lamellar liquid crystal coating.

U.S. Patent No. 8299162 reports a powder comprising a water-soluble wax effective for cold-processing a cream or lotion or gel when mixed with water.

PCT application WO 2005/007242 relates to solvated nonionic surfactants and fatty acids combined with an alkoxylated alkanolamide and water, if needed, to create a liquid and readily flowable composition.

PCT application WO 2008/006697 discloses a method of measuring and of selecting compositions of enhanced post-wash hydration value.

Japanese Patent Application Publication JP 2017/110139 discloses a framed transparent solid soap comprising fatty acid salt, myristyl alcohol, and hydroxypropyl methylcellulose. None of the additional information above describes the combination of partially neutralized fatty acid and high HLB surfactant that provides a link to making a cosmetic composition precursor for hydration or the rehydration of a cosmetic composition precursor to form an end-use composition.

### Summary

The present inventors have developed a cosmetic composition precursor that, unexpectedly, can be easily hydrated with the addition of water, water-miscible solvent or mixture thereof for a customizable cosmetic, end-use formulation.

Accordingly, in a first aspect, the invention relates to a cosmetic composition precursor according to claim 1.

In a second aspect, the present invention is directed to a method of hydrating a cosmetic composition precursor of the first aspect with the addition of water, water-miscible solvent or mixture thereof for a customizable cosmetic, end-use formulation.

In a third aspect, the present invention is directed to an end-use cosmetic composition prepared from the precursor composition as defined in the first aspect wherein further customization is achieved by the optional addition of desired ingredients.

The present disclosure also refers to a method of treating skin in need of treatment comprising the steps of making an end-use composition comprising the precursor composition as defined in the first aspect, and applying the end-use cosmetic composition onto skin in need of treatment.

All other aspects of the present invention will become more readily apparent upon considering the detailed description, data, and examples which follow.

For the avoidance of doubt, the term "comprising" is meant not to be limiting to any stated elements but rather to encompass non-specified elements of major or minor functional importance. Therefore, the listed steps, elements or options need not be exhaustive. Whenever the words "including" or "having" are used, these terms are meant to be equivalent to "comprising" as defined above. All ranges are meant to include all those ranges subsumed therein.

The term "skin" as used herein includes the skin on the face, neck, chest, back, arms, axilla, buttocks, hands, legs and scalp. Skin benefit agent, as used herein, is meant to include a component that (a) improves a facial or body characteristic after topical application like a skin characteristic, (b) benefits the same, or (c) both (a) and (b). As used herein, the terms "formulation" and "composition" are meant to be used to refer to the same ready-to-use mixture of ingredients. End-use composition (preferably water continuous) is a composition for topical application and includes a cream, lotion, balm, serum, gel, deodorant, antiperspirant, nail treatment, make-up and moisturizing bar. In an especially preferred embodiment, the end-use composition of this invention is a leave-on composition. Substantially free of, as used herein, is intended to mean comprising less than 10% by weight. The term "implement," as used herein, includes mixing blades, hands, utensils, spatulas and whisks. Liquid, as used herein, is intended to mean that the substance is a pumpable and/or flowable, preferably, water continuous composition. Reconstitution, as used herein, refers to the process of transforming a solid, including a solid substantially free of water, into a liquid (i.e. lotion or cream) format by adding water to it. The standard used herein for reconstitution time when referring to the phrase "rapid/instant hydration" is over 5 seconds, preferably, from 10 seconds to 3 minutes, and most preferably, from 10 seconds to 2 minutes. In the scope of the present invention, the phrase "high HLB" is used to refer to surfactants with an HLB value that is greater than or equal to 8. "HLB" stands for the hydrophilic-lipophilic balance of a surfactant, used to measure the degree to which a surfactant is hydrophilic or lipophilic, and is widely used in the art. The term "leave-on" as used with reference to compositions herein means a composition that is applied to or rubbed on the skin, and left thereon. The end use compositions of the present have been developed for applications that include non-therapeutic and cosmetic topical applications to skin.

Except in the examples, or where otherwise explicitly indicated, all numbers in this description indicating amounts of material are to be understood as modified by the word "about." All amounts are by total weight of the composition, unless otherwise specified.

### Detailed Description of the Invention

The fatty acid compounds comprised in the cosmetic composition precursor of the present disclosure are C₁₀-C₂₂ fatty acids, preferably, C₁₆-C₁₈ fatty acids, more preferably, 3:1 to 1:3 ratios of C₁₆:C₁₈, even more preferably 1:1 to 1:2 ratios of C₁₆:C₁₈, and still even more preferably 1:1 to 1:1.5 weight ratios of C₁₆:C₁₈ fatty acids. The fatty acid comprises of lauric, myristic, palmitic, stearic, isostearic, hydroxystearic, oleic, linoleic, ricinoleic, arachidic, behenic, erucic acid or mixtures thereof. In a preferred embodiment of the invention, the fatty acid used is stearic acid, palmitic acid or a mixture thereof. The composition comprises 30 to 70%, preferably, 35 to 65%, most preferably, 40 to 60% by weight of the fatty acid based on the total weight of the cosmetic composition precursor.

Traditional buffers or pH modifiers are suitable for inclusion to the cosmetic composition precursors of this invention for partial neutralization of the C₁₀-C₂₂ fatty acids. These include common additives such as inorganic bases (e.g. sodium hydroxide and potassium hydroxide) and organic bases (e.g. triethanolamine and aminomethyl propanol). It is within the scope of the invention to employ both organic and inorganic bases as neutralizing agents. Thus, in a preferred embodiment, inorganic bases, organic bases or a mixture thereof are used. In an especially preferred embodiment, inorganic bases such as sodium hydroxide, potassium hydroxide, or a mixture thereof are used. The inorganic bases partially neutralize the fatty acid compounds comprised in the invention wherein the degree of neutralization is at least 10%, preferably 20 to 65%, more preferably 25 to 60%, and still more preferably 30 to 55%.

High HLB surfactant is present in the cosmetic composition precursor of the present disclosure. Surfactants suitable for use in the present invention have an HLB value greater than or equal to 8, preferably, from 8 to 19, more preferably, 8 to 17, most preferably, 8 to 15. In a particularly preferred embodiment, the high HLB surfactant has an HLB value from 8 to 12. The high HLB surfactants suitable for use include arachidyl glucoside, cetearyl glucoside, coco-glucoside, decyl glucoside, PEG-100 stearate, polysorbate 40, polysorbate 20, steareth-21, PEG-40 hydrogenated castor oil, sucrose cocoate, sucrose stearate or a mixture thereof. These high HLB surfactants may further be provided with fatty alcohols and/or additional fatty acids to aid in lamellar structuring of the end-use composition. Illustrative examples include cetearyl alcohol, cetyl alcohol, stearyl alcohol, isostearyl alcohol, behenyl alcohol, stearic acid, palmitic acid, lauric acid and combinations thereof. In an embodiment of the invention, a blend of arachidyl alcohol, behenyl alcohol, and arachidyl glucoside, as made commercially available from the supplier Seppic under the trade name Montanov^{™} 202 and characterized by an HLB of 8.3, is used as the surfactant. In a particularly preferred embodiment of the invention, a blend of sugar-based cetearyl alcohol and cetearyl glucoside is the surfactant. Cetearyl alcohol and cetearyl glucoside are commercially available as a blend from the supplier Seppic under the tradename Montanov^{™} 68 and is characterized by an HLB value of 9.3. Total concentration of the high HLB surfactant may range from 10 to 70%, preferably 25 to 65%, most preferably 40 to 60% by weight of the cosmetic composition precursor, including all ranges subsumed therein.

In an embodiment of the disclosure, the ratio of fatty acid with at least 10% neutralization to high HLB surfactant is 20:80 to 80:20, most preferably 30:70 to 70:30.

The above ingredients, at the very least, are to be combined and mixed into a uniform composition. In a preferred embodiment, the resulting cosmetic composition precursor comprises of less than 10% by weight water. In another preferred embodiment, the cosmetic composition precursor comprises of 5% by weight or less of water. In still another preferred embodiment, the cosmetic composition precursor comprises of 2.5% by weight or less of water. In yet another preferred embodiment, the cosmetic composition precursor comprises from 0.001 to 2.2% by weight of water.

Optional ingredients that may benefit the consumer including fragrances, emulsifiers, sensory modifiers and skin benefit agents can be included in cosmetic composition precursors of the present invention. Skin benefit agents suitable for use in this invention are meant to include but not be limited to opacifiers, colorants, emollients, occlusive agents, plant extracts, optical agents, skin lightening agents, anti-inflammatory agents, anti-acne agents, sunscreens, photostabilizers, humectants, wrinkle reducing agents, desquamation promoters, exfoliating agents, mixtures thereof or the like. Each of these substances may range from 0.05 to 15%, preferably between 0.1 and 10%, most preferably between 0.2 to 5% by weight of the total weight of the cosmetic composition precursor.

The cosmetic composition precursor of the present invention can be sieved or subjected to any of the conventional methods known to those skilled in the art capable of rendering the precursor mixture into a powder form to facilitate subsequent hydration processes. In an embodiment of the invention, the cosmetic composition precursor powder is in a powdered or flake form. In another embodiment of the invention, the cosmetic composition precursor has a particle size range of 0.5 to 200 µm, preferably 5 to 150 µm. In a particularly preferred embodiment of the invention, the particle size range is 10 to 50 µm. The particle size can be assessed by techniques known to those skilled in the art such as a Leitz optical microscope (e.g. ones of the Laborlux 12 Pol S model name) fitted with cross-polarizers under magnification settings of 5x to 50x.

The cosmetic composition precursor is then to be hydrated by combining the precursor with water, a water-soluble solvent or mixture thereof to make the desired end use composition. Accordingly, the cosmetic composition precursor to be hydrated can be used at inclusion levels from 1 to 20%, preferably, 2 to 17%, and more preferably, 3 to 15% by weight of the total end-use composition. Water is the preferred solvent to be used in the hydration of the cosmetic composition precursor. It is within the scope of the invention to hydrate the cosmetic composition precursor at water temperatures ranging from 3°C to 60°C, preferably, 5°C to 55°C, and more preferably, 7°C to 50°C. Amount of water may range from 60 to 99%, preferably, from 70 to 95%, and more preferably, from 75 to 90% based on the total weight of the end-use composition.

Viscosity of the end-use composition of this invention is typically from about 1,000 to about 120,000 cps, preferably, about 2,000 to 100,000, and most preferably, from about 5,000 to 80,000 cps, taken under conditions of 25°C and a shear rate of 1s⁻¹ with a strain controlled parallel plate rheometer made commercially available from suppliers like T.A. Instruments under the Discovery name. Alternatively, viscosity can also be measured using a Brookfield Viscometer (speed at 20 rpm, spindle 5, Helipath off, for one (1) minute at 25°C).

It is also possible to characterize the hydrated end-use composition by measurement of its pH. In the present invention, the pH of the cosmetic composition precursor is from 3 to 9, preferably, from 4 to 8.5, and most preferably, from 5 to 8. pH may be measured by use of a pH meter, such as the Orion Star A111 Benchtop pH meter made commercially available from Thermo Scientific.

In another embodiment herein, the present invention encompasses a method of hydrating the cosmetic composition precursor to result in an end-use cosmetic composition wherein further customization is achieved by the optional addition of desired ingredients. The method comprises the steps of combining, in no particular order, 1 to 20%, preferably, 2 to 17%, and more preferably, 3 to 15% of the cosmetic composition precursor with the desired solvent preferably using a reusable and/or nondisposable container of choice, such as a bowl or a jar with a secure lid; mixing with an implement or applying shear or agitating the contents in a moderate fashion for at least 10 seconds until uniform; and allowing the resulting agitated mixture to sit still for at least 10 seconds, preferably, at least 15 seconds, most preferably, at least 30 seconds to produce the desired hydrated end-use composition. In an embodiment of the invention, the end use composition of the present invention is ready for use within 10 seconds to 3 minutes after making. Suitable solvents employed in the invention include water, sea water, rosewater (e.g. *Rosa Damascena* flower Water), tea (e.g. *Camellia Sinensis* Leaf Water), *Aloe Barbadensis* (aloe vera) leaf juice, witch hazel (e.g. *Hamamelis Virginiana* extract), lavender water (e.g. *Lavendula Angustifolia* flower water), grape water (e.g. *Vitis Vinifera* fruit water and *Vitis Vinifera* juice) or mixtures thereof. In a particularly preferred embodiment, the solvent is water. The cosmetic composition precursor is instantly hydratable in the solvent of choice and is characterized by a rapid build in structure immediately following agitation. It is possible to further customize the end-use cosmetic composition as desired by, for example, optionally adding in sensory modifiers, emollients, humectants, preservatives and skin benefit agents. In still another embodiment, if consumers were to hydrate the cosmetic composition precursor on demand, the end-use composition can be made to be preservative-free.

In yet another embodiment herein, the present invention encompasses a method of hydrating the cosmetic composition precursor to result in an end-use cosmetic composition wherein skin benefit agents, fragrance and preservatives may be added to the composition of the precursor itself. The method comprises the steps of dispensing a desired amount of precursor in the palm of one's hand, adding water to activate said precursor directly or applying said precursor onto a wet surface, shearing the precursor and water together (e.g. pressure of palms pressed against each other) with moderate force until uniform. It is possible to reverse the first and second steps above by, for example, instead first adding water to one's palm and then dispensing a desired amount of precursor.

In still another embodiment herein, the present invention encompasses a method of sustainably transporting in the absence of a liquid, such as water, a water-miscible solvent or mixture thereof a cosmetic composition precursor that can be further processed at another location, such as a consumer's home or a factory, in an environmentally friendly and potentially plastic-free manner (e.g. using paperboard to package the cosmetic composition precursor). The method comprises the steps of producing the cosmetic composition precursor according to the present invention; packing the cosmetic composition precursor in presence of no or substantially free of liquids; transporting the cosmetic composition precursor to one's home or a manufacturing site by any conventional means known to one skilled in the art; hydrating the cosmetic composition precursor with solvent of choice present at home or the manufacturing site under appropriate agitation; and adding in optional ingredients, in no particular order, to create the end-use composition. This method allows for a more compact and economical shipping and delivery process wherein the solvent is not involved in the transportation procedure.

Upon hydration of the cosmetic composition precursor, one now may add in the aforementioned optional ingredients to the end-use composition in lieu of directly to the cosmetic composition precursor. These optional ingredients may be added during manufacturing or by the consumer, depending on factors such as accessibility of ingredients. Each of these substances may range from 0.05 to 25%, preferably between 0.1 and 2% by weight of the end-use composition.

Silicones are suitable for optional use in the present invention and may be categorized into the volatile and nonvolatile variety. Amounts may range, for example, from 0.01 to 25%, more preferably from 0.1 to 20%, and most preferably, from 0.5 to 6% by weight of the end-use composition. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Nonvolatile silicones useful in the end-use composition include polyalkyl siloxanes, polyalkylaryl siloxanes and polyether siloxane copolymers. The essentially nonvolatile polyalkyl siloxanes useful herein include, for example, polydimethyl siloxanes with viscosities from about 5 x 10⁻⁶ to 0.1 m²/s at 25°C. Emulsifying and non-emulsifying silicone elastomers are also suitable for use in the end-use composition of this invention.

Conventional humectants, generally of the polyhydric alcohol-type materials, may be used in the present invention. Typical polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The most preferred embodiment includes glycerin, propylene glycol or a mixture thereof. The amount of humectant employed may range anywhere from 0.1 to 25%, preferably, between 0.5 and 20%, and more preferably, between 1 and 15% by weight of the end-use composition.

Triglycerides are another group of materials suitable for optional use in the present invention. Illustrative but not limiting examples are sunflower seed oil, cotton oil, canola oil, grapeseed oil, soybean oil, castor oil, borage oil, olive oil, shea butter, jojoba oil and mixtures thereof. Mono- and di-glycerides may also be useful. Illustrative of these categories are glyceryl monostearate and glyceryl distearate. Amounts of triglycerides may range from 0.01 to 10%, preferably from 0.1 to 8% by weight of the end-use composition.

While the end-use composition has sufficient stability and does not require the use of additional emulsifiers, in an embodiment of the invention, the high HLB surfactants may be mixed with optional low or high HLB emulsifiers. The emulsifier may be selected from the group consisting of anionic, nonionic, cationic and amphoteric surfactants. In a preferred embodiment, emulsifiers used are of the nonionic or cationic type, or a mixture thereof. In another preferred embodiment of the invention, low HLB surfactants may optionally be added, wherein the combined HLB will optionally be at least 8. It is within the scope of the present invention to use surfactants with a low HLB value, provided that the combined HLB value overall is greater than or equal to 8. The overall HLB of the emulsifier or emulsifiers used when water-continuous emulsions are desired will be 8 to 17, and preferably, from 8.5 to 15, and most preferably from 9 to 14.

The nonionic emulsifiers suitable for use can include any of the range of nonionic emulsifiers known to one of ordinary skill in the art. Particularly preferred nonionic surfactants are those with a C₁₀-C₂₀ fatty alcohol or acid hydrophobe condensed with from about 2 to about 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic emulsifiers. Still other preferred nonionic surfactants include glyceryl stearate, glycol stearate and stearamide AMP.

Preferred cationic emulsifiers that may be used include, for example, palmitamidopropyltrimonium chloride, distearyldimonium chloride, diester quaternary ammonium compounds (e.g. distearoylethyl dimonium chloride) and mixtures thereof.

Total concentration of the emulsifier may range from 0.1 to 20%, and preferably, from 1 to 10%, and most preferably, from 1 to 8% by weight of the end-use composition, including all ranges subsumed therein.

If desired, anionic and amphoteric emulsifiers can also be optionally added. Some preferred anionic emulsifiers can include alkyl ether sulfate and sulfonates, alkyl sulfates and sulfonates, alkylbenzene sulfonates, alkyl and dialkyl sulfosuccinates, C₈-C₂₀ acyl isethionates, C₈-C₂₀ acyl methyl isethionates, C₈-C₂₀ acyl methyl taurates, C₈-C₂₀ alkyl ether phosphates, alkyl ether carboxylates and combinations thereof.

Useful amphoteric emulsifiers can include cocoamidopropyl betaine, C₁₂-C₂₀ trialkyl betaines, sodium lauroamphoacetate, and sodium laurodiamphoacetate or a mixture thereof.

Emulsifying/thickening additives include crosslinked acrylates (e.g. Carbopol^{®} 980), hydrophobically-modified acrylates (e.g. Carbopol^{®} 1382), polyacrylamides (e.g. Sepigel ^{™} 305), acryloylmethylpropane sulfonic acid/salt polymers and copolymers (e.g. Aristoflex^{®} HMB and AVC), cellulosic derivatives and natural gums, starches and mixtures thereof can also be optionally added. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums.

Hydrocarbons may be optionally included in the end-use composition of the present invention. Suitable hydrocarbons may include mineral oil, petrolatum and polyalphaolefins. Examples of preferred volatile hydrocarbons include polydecanes such as isodecane and isododecane and the C₇-C₈ through C₁₂-C₁₅ isoparaffins.

End-use compositions of the present invention may include vitamins as the desired additive, including derivatives thereof such as vitamin alkyl esters. Illustrative vitamins are vitamin A, vitamin B₂, vitamin B₃ (niacinamide), vitamin B₅ (panthenol), vitamin B₆, vitamin C, vitamin E, vitamin K, folic Acid and biotin. Derivatives of the vitamins may also be employed. For instance, vitamin C derivatives include ascorbyl tetraisopalmitate, magnesium ascorbyl phosphate and ascorbyl glucoside. Derivatives of vitamin E include tocopheryl acetate, tocopheryl palmitate and tocopheryl linoleate. Derivatives of vitamin B₃ include nicotinic acid. Total amount of vitamins when present in cosmetic compositions according to the present invention may range from 0.001 to 10%, preferably from 0.01 to 5%, optimally from 0.05 to 2.5% by weight of the end-use composition.

Other additional optional skin benefit agents suitable for use in this invention include minerals and skin nutrients such as milk; magnesium, calcium, copper, zinc and other metallic components; retinoic acid and its derivatives (e.g., cis and trans); retinal; retinol; retinyl esters such as retinyl acetate, retinyl palmitate, and retinyl propionate; alpha hydroxy acids, beta hydroxy acids, e.g. salicylic acid and derivatives thereof (such as 5-octanoyl salicylic acid, heptyloxy 4 salicylic acid, and 4-methoxy salicylic acid); resorcinol derivatives (particularly 4-substituted resorcinol derivatives, including 4-ethyl resorcinol, 4-isopropyl resorcinol, 4-hexyl resorcinol, 4-cyclopentyl resorcinol, 4-cyclohexyl resorcinol and acylated forms thereof); resveratrol, saccharide isomerate, ceramides (e.g. Ceramide 1, Ceramide 3, Ceramide 3B and Ceramide 6) and pseudoceramides, allantoin, pentaerythrityl tetra-di-t-butyl hydroxyhydrocinnamate, pyroglutamic acid (PCA) salt derivatives including zinc PCA and sodium PCA, 12-hydroxystearic acid, petroselinic acid, conjugated linoleic acid, octadecanoic acid, hyaluronic acid and its salt derivatives mixtures thereof and the like. Such skin benefit agents, when used, collectively make up from 0.001 to 12% by weight of the end-use composition.

A wide selection of botanical extracts may optionally be included in end-use compositions of this invention. The extracts may either be soluble in water or oil, carried in a solvent that is hydrophilic or hydrophobic, respectively. In the preferred embodiment, water or ethanol are the extract solvents. Illustrative examples include those extracted from green tea, yarrow, chamomile, licorice, aloe vera, citrus unshui, willow bark, alfalfa, algae, witch hazel, sage, thyme and rosemary, as well as oils such as those derived from sea buckthorn, moringa, argan, avocado, calendula, algal and marula. Soy extracts may be used and especially when it is desirable to include retinol. Such extracts, when used, are preferably employed in collective amounts ranging from 0.001 to 12% by weight of the end-use composition.

Another optional additive suitable for use includes hemp oil with 2.5 to 25% by weight cannabigerol and/or cannabidiol at from 0.5 to 10 percent by weight. When used, such oil makes up from 0.0001 to 12% by weight of the composition, and preferably, from 0.01 to 5% by weight of the end-use composition, including all ranges subsumed therein.

Preservatives can be incorporated into the end-use compositions of this invention as desired to protect against the growth of potentially harmful microorganisms. Cosmetic chemists are familiar with appropriate preservatives and routinely choose them to satisfy preservative tests and product stability tests. Preservative systems should be selected having regard for the use of the composition and possible incompatibilities between the preservatives and other ingredients in the formulation. Exemplary examples of preservatives for compositions of this invention include, without limitations, iodopropynyl butyl carbamate (IPBC), phenoxyethanol, ethylhexylglycerine, 1,2-octanediol, methyl paraben, propyl paraben, imidazolidinyl urea, sodium dehydroacetate, propanediol, alkyl esters of para-hydroxybenzoic acid, hydroxyacetophenone, DMDM hydantoin derivatives, climbazole, propionate salts, and a variety of quaternary ammonium compounds. Preservatives are preferably employed in amounts ranging from 0.01 to 2% by weight of the end-use composition.

Many types of packaging can be used to store and deliver the end-use composition of the present invention. The selection of packaging is dependent upon the personal care end-use and the viscosity of the composition itself. As an example, leave-on lotions and creams for skin typically employ plastic containers with an opening at a dispense end covered by an appropriate closure. Conventional closures include flip-top hinged lids and screw-caps. In general, patches, bottles, tubes, roller-ball applicators, squeeze containers or lidded jars are preferred.

The present invention as an end-use cosmetic composition is a composition suitable for topical application to human skin, in particular leave-on products, to deliver a skin benefit.

The invention will now be described below in the context of specific non-limiting examples to facilitate a greater understanding of the present invention. One of ordinary skill in the art will recognize that variations of the present invention that differ from the examples given may be practiced without deviating from the teachings of the appended claims.

### Examples

All samples were made by mixing the mentioned ingredients under conditions of moderate sheer, about 22°C to about 85°C, and atmospheric pressure.

### Example 1. Main composition of cosmetic composition precursor

| Cosmetic Composition Precursor Components | HLB value | High HLB surfactants (wt.%) | | | | 5. Low HLB surfactants (wt. %) | 6. No KOH (wt.%) |
|---|---|---|---|---|---|---|---|
| | | Sugar-based | | Ethoxylated | | | |
| | | 1. | 2. | 3. | 4. | | |
| Stearic Acid | - | 51.4 | 51.4 | 51.4 | 51.4 | 51.4 | 51.4 |
| Glycol Stearate & Stearamide AMP | - | - | - | - | - | 25.7 | - |
| Glyceryl Stearate | 3.8 | - | - | - | - | 12.0 | - |
| Cetyl Alcohol | - | - | - | - | - | 6.8 | - |
| Cetearyl Alcohol | - | - | - | 31.2 | 31.2 | - | - |
| Montanov^{™} 68 | 9.3 | 44.5 | - | - | - | - | 48.6 |
| Montanov^{™} 202 | 8.3 | - | 44.5 | - | - | - | - |
| PEG-100 Stearate | 18.8 | - | - | 13.3 | - | - | - |
| Polysorbate 40 | 15.6 | - | - | - | 13.3 | - | - |
| 45% KOH Solution | - | 4.10 | 4.10 | 4.10 | 4.10 | 4.10 | 0.00 |
| Reconstitution (<1 min), where 6% precursor was mixed with 94% water | | <1 minute | <1 minute | <1 minute | <1 minute | ~ 10 minutes | > 10 min |

Samples 1 and 2 illustrate sugar-based surfactants with high HLB values. Samples 3 and 4 both demonstrate ethoxylated surfactants with high HLB values. Sample 5 illustrates low HLB surfactants. The composition of sample 6 is the same as that of sample 1, but without the inclusion of any base.

The results demonstrate the unexpected importance of using high HLB surfactants in the cosmetic composition precursor in considering reconstitution time. In samples with high

HLB surfactants, it is shown that reconstitution requires less than 1 minute, whereas sample 5, with a low HLB surfactant, requires approximately 10 minutes. Further, the results illustrate that a sample with high HLB surfactant but lacks an inorganic base to achieve at least 10% neutralization of the fatty acid, such as potassium hydroxide, does not allow for a fast reconstitution time. In the case of sample 6, where fatty acid is not neutralized, it requires a duration over 10 minutes.

### Example 2. Acid-Soap Ratio for Montanov^{™} 68/Stearic Acid system

| **Acid-Soap Ratio for Montanov^{™} 68/Stearic Acid System** | | | | | | | |
|---|---|---|---|---|---|---|---|
| | **0% SA neut.** | **6% SA neut.** | **13% SA neut.** | **18% SA neut.** | **23% SA neut.** | **33% SA neut.** | **44% SA neut.** |
| Stearic Acid | 55.5% | 54.0% | 52.5% | 51.4% | 50.5% | 48.5% | 46.5% |
| Montanov^{™} 68 | 44.5% | 44.5% | 44.5% | 44.5% | 44.5% | 44.5% | 44.5% |
| 45% KOH Solution | 0.00% | 1.50% | 3.00% | 4.10% | 5.00% | 7.00% | 9.00% |
| Reconstitution (< 1 min) | No | No | Yes | Yes | Yes | Yes | Yes |

The results show that stearic acid having a partial neutralization of at least 10% in a cosmetic composition precursor made according to the present invention unexpectedly results in a reconstitution time under 1 minute.

### Example 3. Impact of different counterions in Montanov^{™} 68/Stearic Acid system

| | **18% SA neut.** | **18% SA neut.** | **18% SA neut.** |
|---|---|---|---|
| Stearic Acid | 51.4% | 51.4% | 51.4% |
| Montanov^{™} 68 | 44.5% | 46.0% | 43.6% |
| 45% KOH Solution | 4.10% | - | - |
| 50% NaOH Solution | - | 2.60% | - |
| 99% TEA Solution | - | - | 5.00% |
| Reconstitution (< 1 min) | Yes | Yes | Yes |

The results demonstrate that, notwithstanding the type of counterion, it is suitable for the cosmetic composition precursor to include a neutralizing base of differing counterions without impacting or compromising the rate of instant hydration of the resulting cosmetic composition precursor. Each sample in Example 3 resulted in reconstitution times within 1 minute.

### Example 4. Use of cosmetic composition precursor to create a customized end-use lotion

| Ingredients | % | Function |
|---|---|---|
| Water | 78.1 | Base |
| Precursor | 5 | Base |
| Glycerin | 5 | Humectant |
| Elastomer | 10 | Sensory modifier |
| Retinol (15% solution in caprylic/capric triglyceride) | 0.7 | Skin benefit agent |
| 4-hexylresorcinol (20% solution in caprylic/capric triglyceride) | 1.2 | Skin benefit agent |

This example is illustrative of using the cosmetic composition precursor of the present invention to create an end-use composition containing a humectant, sensory modifier, and desired skin benefit agents. The end-use composition made had a stable viscosity and no observed syneresis.

## Claims

1. A cosmetic composition precursor comprising:
(a) C₁₀₋₂₂ fatty acid; and
(b) high HLB surfactant,
wherein the HLB value is at least 8 and wherein the C₁₀₋₂₂ fatty acid is partially neutralized at least 10%, further wherein the ratio of fatty acid to high HLB surfactants is between 10:90 to 90:10; wherein the fatty acid comprises lauric, myristic, palmitic stearic, isostearic, hydroxystearic, oleic, linoleic, ricinoleic, arachidic, behenic, erucic acids or mixture thereof, and further wherein the precursor comprises 30 to 70% by weight of the fatty acid; wherein the high HLB surfactant comprises arachidyl glucoside, cetearyl glucoside, coco-glucoside, decyl glucoside, PEG-100 stearate, polysorbate 40, polysorbate 20, steareth-21, PEG-40 hydrogenated castor oil, sucrose cocoate, sucrose stearate or mixtures thereof, and further wherein the precursor comprises 10 to 70% by weight of the surfactant.

2. The cosmetic composition precursor according to claim 1 wherein the fatty acid is stearic acid, palmitic acid, lauric acid or a mixture thereof.

3. The cosmetic composition precursor according to claim 1 wherein the high HLB surfactant comprises cetearyl glucoside or is in combination with a fatty alcohol.

4. A method of hydrating the cosmetic composition precursor according to any one of claims 1 to 3 with water, a water-miscible solvent or mixture thereof comprising the steps of:
(a) adding 1 to 20% of the precursor to water, a water-miscible solvent or mixture thereof to a container;
(b) mixing with an implement or agitating for at least 10 seconds until a uniform mixture is produced;
(c) allowing the mixture to sit still for at least 10 seconds or using the agitated mixture immediately if the implement is a pair of hands;
(d) optionally adding in sensory modifiers, emollients, humectants, preservatives, fragrances, skin benefit agents or a mixture thereof.

5. A method of hydrating the cosmetic composition precursor according to any one of claims 1 to 3 with water, a water-miscible solvent or mixture thereof comprising, in no particular order, the steps of:
(a) dispensing 1 to 20% of the precursor into a palm of one's hand;
(b) adding water, a water-miscible solvent or mixture thereof to hydrate said precursor;
(c) shearing the precursor and water, a water-miscible solvent or mixture thereof together until a uniform mixture is produced,
wherein steps (a) and (b) may be reversed in relative sequence to each other.

6. A method of sustainably transporting a cosmetic composition precursor in a manner that is substantially free of liquids and packaging comprising, in no particular order, the steps of:
(a) producing or obtaining the cosmetic composition precursor according to according to any one of claims 1 to 3 ;
(b) packing the precursor in absence of any liquids;
(c) transporting the precursor to a manufacturing site;
(d) hydrating the precursor at the manufacturing site with water under mixing or agitation;
(e) optionally adding in additional ingredients to create an end-use composition.

## Patentansprüche

1. Vorläufer einer kosmetischen Zusammensetzung, umfassend:
(a) C₁₀₋₂₂-Fettsäure; und
(b) Tensid mit hohem HLB-Wert,
wobei der HLB-Wert mindestens 8 beträgt und wobei die C₁₀₋₂₂-Fettsäure zu mindestens 10% teilweise neutralisiert ist, wobei ferner das Verhältnis von Fettsäure zu Tensiden mit hohem HLB-Wert zwischen 10:90 bis 90:10 liegt, wobei die Fettsäure Laurin-, Myristin-, Palmitin-, Stearin-, Isostearin-, Hydroxystearin-, Öl-, Linol-, Ricinol-, Arachidin-, Behen-, Erucasäuren oder eine Mischung davon umfasst und wobei ferner der Vorläufer 30 bis 70 Gewichts-% der Fettsäure umfasst; wobei das Tensid mit hohem HLB-Wert Arachidylglucosid, Cetearylglucosid, Coco-Glucosid, Decylglucosid, PEG-100-Stearat, Polysorbat 40, Polysorbat 20, Steareth-21, PEG-40-hydriertes Rizinusöl, Saccharosecocoat, Saccharosestearat oder Mischungen davon umfasst und wobei ferner der Vorläufer 10 bis 70 Gewichts-% des Tensids umfasst.

2. Vorläufer einer kosmetischen Zusammensetzung nach Anspruch 1, wobei die Fettsäure Stearinsäure, Palmitinsäure, Laurinsäure oder eine Mischung davon ist.

3. Vorläufer einer kosmetischen Zusammensetzung nach Anspruch 1, wobei das Tensid mit hohem HLB-Wert Cetearylglucosid umfasst oder in Kombination mit einem Fettalkohol steht.

4. Verfahren zum Hydratisieren des Vorläufers einer kosmetischen Zusammensetzung nach irgendeinem der Ansprüche 1 bis 3 mit Wasser, einem mit Wasser mischbaren Lösungsmittel oder einer Mischung davon, umfassend die Schritte:
(a) Zugabe von 1 bis 20% des Vorläufers zu Wasser, einem mit Wasser mischbaren Lösungsmittel oder einer Mischung davon in einen Behälter;
(b) mindestens 10 Sekunden lang Mischen mit einem Hilfsmittel oder Rühren, bis eine gleichmäßige Mischung hergestellt ist;
(c) Ruhenlassen der Mischung für mindestens 10 Sekunden oder unmittelbares Verwenden der gerührten Mischung, wenn es sich bei dem Hilfsmittel um ein Paar Hände handelt;
(d) optional Hinzufügen von sensorischen Modifikatoren, Erweichungsmitteln, Feuchthaltemitteln, Konservierungsmitteln, Duftstoffen, Hautpflegemitteln oder einer Mischung davon.

5. Verfahren zum Hydratisieren des Vorläufers einer kosmetischen Zusammensetzung nach irgendeinem der Ansprüche 1 bis 3 mit Wasser, einem mit Wasser mischbaren Lösungsmittel oder einer Mischung davon, das in keiner bestimmten Reihenfolge die folgenden Schritte umfasst:
(a) Dispergieren von 1 bis 20% des Vorläufers in die Handfläche;
(b) Zugeben von Wasser, eines mit Wasser mischbaren Lösungsmittels oder einer Mischung davon, um den Vorläufer zu hydratisieren;
(c) gemeinsames Scheren des Vorläufers und des Wassers, eines mit Wasser mischbaren Lösungsmittels oder einer Mischung davon, bis eine gleichmäßige Mischung hergestellt ist,
wobei die Schritte (a) und (b) in relativer Reihenfolge zueinander umgekehrt ablaufen können.

6. Verfahren zum nachhaltigen Transportieren eines Vorläufers einer kosmetischen Zusammensetzung in einer Weise, die im Wesentlichen frei von Flüssigkeiten und Verpackung ist, umfassend, in keiner bestimmten Reihenfolge, die Schritte:
(a) Herstellen oder Erhalt des Vorläufers der kosmetischen Zusammensetzung nach irgendeinem der Ansprüche 1 bis 3;
(b) Verpacken des Vorläufers in Abwesenheit jeglicher Flüssigkeiten;
(c) Transportieren des Vorläufers zu einer Produktionsstätte;
(d) Hydratisieren des Vorläufers in der Produktionsstätte mit Wasser unter Mischen oder Rühren;
(e) optionales Hinzufügen weiterer Bestandteile, um eine Zusammensetzung für die Endverwendung zu erzeugen.

## Revendications

1. Précurseur de composition cosmétique comprenant:
(a) un acide gras en C₁₀₋₂₂; et
(b) un tensioactif à HLB élevée,
dans lequel la valeur HLB est d'au moins 8 et dans lequel l'acide gras en C₁₀₋₂₂ est partiellement neutralisé à au moins 10 %, en outre dans lequel le rapport de l'acide gras aux tensioactifs à HLB élevée est entre 10:90 et 90:10; dans lequel l'acide gras comprend les acides laurique, myristique, palmitique, stéarique, isostéarique, hydroxystéarique, oléique, linoléique, ricinoléique, arachidique, béhénique, érucique ou un mélange de ceux-ci, et dans lequel en outre le précurseur comprend 30 à 70 % en poids de l'acide gras; dans lequel le tensioactif à HLB élevée comprend de l'arachidyl glucoside, du cétéaryl glucoside, du coco-glucoside, du décyl glucoside, du stéarate de PEG-100, du polysorbate 40, du polysorbate 20, du stéareth-21, de l'huile de ricin hydrogénée PEG-40, du cocoate de saccharose, du stéarate de saccharose ou des mélanges de ceux-ci, et en outre dans lequel le précurseur comprend 10 à 70 % en poids du tensioactif.

2. Précurseur de composition cosmétique selon la revendication 1, dans lequel l'acide gras est l'acide stéarique, l'acide palmitique, l'acide laurique ou un mélange de ceux-ci.

3. Précurseur de composition cosmétique selon la revendication 1, dans lequel le tensioactif à HLB élevée comprend du cétéaryl glucoside ou est en combinaison avec un alcool gras.

4. Procédé d'hydratation du précurseur de composition cosmétique selon l'une quelconque des revendications 1 à 3 avec de l'eau, un solvant miscible à l'eau ou un mélange de ceux-ci comprenant les étapes de:
(a) ajouter 1 à 20 % du précurseur à de l'eau, un solvant miscible à l'eau ou un mélange de ceux-ci dans un récipient;
(b) mélanger avec un instrument ou agiter pendant au moins 10 secondes jusqu'à ce qu'un mélange uniforme soit produit;
(c) laisser reposer le mélange pendant au moins 10 secondes ou utiliser le mélange agité immédiatement si l'instrument est une paire de mains;
(d) éventuellement ajouter des modificateurs sensoriels, des émollients, des humectants, des conservateurs, des parfums, des agents bénéfiques pour la peau ou un mélange de ceux-ci.

5. Procédé d'hydratation du précurseur de composition cosmétique selon l'une quelconque des revendications 1 à 3 avec de l'eau, un solvant miscible à l'eau ou un mélange de ceux-ci comprenant, sans ordre particulier, les étapes de:
(a) distribuer 1 à 20 % du précurseur dans une paume de main d'une personne;
(b) ajouter de l'eau, un solvant miscible à l'eau ou un mélange de ceux-ci pour hydrater ledit précurseur;
(c) cisailler le précurseur et l'eau, un solvant miscible à l'eau ou un mélange de ceux-ci jusqu'à ce qu'un mélange uniforme soit produit,
dans lequel les étapes (a) et (b) pouvant être inversées dans leur séquence relative l'une par rapport à l'autre.

6. Procédé de transport durable d'un précurseur de composition cosmétique d'une manière sensiblement exempte de liquides et d'emballage comprenant, sans ordre particulier, les étapes de:
(a) produire ou obtenir le précurseur de composition cosmétique selon l'une quelconque des revendications 1 à 3;
(b) emballer le précurseur en l'absence de tout liquide;
(c) transporter le précurseur jusqu'à un site de fabrication;
(d) hydrater le précurseur sur le site de fabrication avec de l'eau sous mélange ou agitation;
(e) éventuellement ajouter des ingrédients supplémentaires pour créer une composition d'utilisation finale.
